# EUROPEAN PATENT SPECIFICATION

(11) **EP 1 966 376 B1**
(45) Date of publication and mention of the grant of the patent: **02.12.2015**
(21) Application number: 06806631.5
(22) Date of filing: 27.10.2006
(51) Int. Cl.: C12N 15/11, A61K 31/7125

(54) **PREVENTION OF RETROVIRAL INFECTIVITY THROUGH INTERFERENCE WITH PPT**
VERHINDERUNG RETROVIRALER INFEKTIOSITÄT DURCH INTERFERENZ MIT PPT
PREVENTION DE L'INFECTIOSITE RETROVIRALE PAR L'INTERFERENCE AVEC LA PPT

(30) Priority: 27.10.2005 EP 05090299; 03.11.2005 EP 05090303; 12.05.2006 EP 06090079
(43) Date of publication of application: 10.09.2008
(73) Proprietor: Mölling, Karin, 14195 Berlin (DE)
(72) Inventor: Mölling, Karin, 14195 Berlin (DE)
(74) Representative: Moré, Solveig Helga
(86) International application number: PCT/EP2006/010462
(87) International publication number: WO 2007/048644

(56) References cited:
- US-A- 5 849 900
- JENDIS J ET AL: "INHIBITION OF REPLICATION OF FRESH HIV TYPE 1 PATIENT ISOLATES BY APOLYPURINE TRACT-SPECIFIC SELF-COMPLEMENTARY OLIGODEOXYNULEOTIDE" AIDS RESEARCH AND HUMAN RETROVIRUSES, MARY ANN LIEBERT, US, vol. 12, no. 12, 1996, pages 1161-1168, XP002930541 ISSN: 0889-2229 cited in the application
- JENDIS JORG ET AL: "Inhibition of replication of drug-resistant HIV type 1 isolates by polypurine tract-specific oligodeoxynucleotide TFO A" AIDS RESEARCH AND HUMAN RETROVIRUSES, vol. 14, no. 11, 20 July 1998 (1998-07-20), pages 999-1005, XP009087386 ISSN: 0889-2229 cited in the application
- MATSKEVICH ALEXEY A ET AL: "Short partially double-stranded oligodeoxynucleotide induces reverse transcriptase/RNase H-mediated cleavage of HIV RNA and contributes to abrogation of infectivity of virions" AIDS RESEARCH AND HUMAN RETROVIRUSES, vol. 22, no. 12, December 2006 (2006-12), pages 1220-1230, XP002444490 ISSN: 0889-2229
- MATZEN K ET AL: "RNase H-mediated retrovirus destruction in vivo triggered by oligodeoxynucleotides" NATURE BIOTECHNOLOGY 2007 UNITED STATES, vol. 25, no. 6, 2007, pages 669-674, XP002444491 ISSN: 1087-0156 1546-1696

## Description

The present invention concerns the inactivation of viral infectivity in a cell-free environment, a pharmaceutical agent for use for the protection of a female from HIV-1 infection during sexual intercourse for application on the vaginal mucosa, as claimed.

### BACKGROUND OF THE INVENTION

Retroviruses like the Human immunodeficiency virus (HIV)-1 continue to be a global pandemic of enormous consequence to humanity. Current standard-of-care regimens recommended for the treatment of HIV infection include two or more nucleos(t)ide reverse transcriptase inhibitors (NRTI) in combination with non-nucleoside reverse transcriptase or protease inhibitor. Importantly, NRTIs are activated through interactions with the cellular machinery for regulating endogenous nucleoside triphosphate pool.

Protease inhibitors also act inside of infected cells. Although some successes have been demonstrated, many agents in these classes are limited by various factors, including insufficient efficiency, poor tolerability, dependency on cellular enzymes and drug-resistance. A major focus of the search for novel strategies to fight HIV-1 is therefore identification antiviral compounds, which would allow overcoming these limitations.

Known, commonly available anti-viral drugs are also targeting three viral proteins, the Reverse Transcriptase (RT), the protease, and gp41. New antiviral approaches are required, to complement available therapeutics.

Retroviruses replicate the viral RNA by concerted action of the reverse transcriptase (RT) and RNase H, which specifically hydrolyzes RNA in RNA-DNA hybrids (Hansen et al. 1988, EMBO J. 7:239-243; Moelling et al., Nature New Biol. 1971, 234:240-243; Tisdale et al., 1991, J. Gen. Virol. 72:59-66.). Thereby the polypurine tract (PPT) resists to hydrolysis and serves as primer for the second strand DNA synthesis (Volkmann et al., 1995, Nucleic Acids Res. 23:1204-1212; Erratum in: Nucleic Acids Res. 23 3804; Wohrl, B.M. and K. Moelling. 1990, Biochemistry 29:10141-10147; Wohrl, et al., 1991, J. Mol. Biol. 220:801-818.). The PPT is one of the most highly conserved sequences of HIV, which is located in the coding region of the nef gene, adjacent to the unique region at the 3'-end (U3) (Martinez et al., 2002, Cell 110: 563-74). The extended PPT is 25 nucleotides long and consists of two polypurine clusters interrupted by two non-purines (CU), which are located adjacent to an internal A. 5' of this ACU triplet is the cleavage site of the viral RNase H (Wohrl, B.M., and K. Moelling. 1990, Biochemisty 29:10141-10147). Furthermore, the transcripts of this sequence are important at later stages during replication for recognition by the integrase and integration of the DNA provirus (Reicin et al., 1995, J. Virol. 69:5904-5907).

Thus, ACU is a sequence of extreme functional importance for three viral enzymes, the RT, the RNase H, and the integrase. The distance between the two active centers of the RT and the RNase H is about 18 nucleotides (Sarafianos et al., 1996, EMBO J. 20:1449-1461; Wohrl, B.M., and K. Moelling. 1990, Biochemistry 29:10141-10147). The PPT has the unique capacity to resist RNase H-mediated hydrolysis of the RNA in DNA-RNA heteroduplexes (Müller et al., 1994, J. Mol. Biol. 242:422-429.), probably due to structural peculiarities (Sarafianos et al., 1996, EM-BO J. 20:1449-1461; Xiong, Y., and M. Sundaralingam, 1998, Structure 6:1493-1501). The PPT-RNA remains hybridized to minus-strand DNA during reverse transcription and functions as primer for initiation of plus-strand DNA synthesis (Volkmann et al., 1995, Nucleic Acids Res. 23:1204-1212, Erratum in: Nucleic Acids Res. 23 3804). This is a preferred recognition site of the RT/RNase H.

The US 5,849,900 discloses oligodeoxynucleotides (ODN) and oligoribonucleotides (ORN) against the polypurine-tract (PPT) of HIV which were designed for antiviral therapy of an HIV- infected individual. The effects of the disclosed ODNs were demonstrated through premature termination of cDNA synthesis at the PPT.

The effect has been shown to be extremely sequence-specific. Three viral enzymes are required to function correctly at the PPT, therefore this site is considered to be preferred for intervention because of a low chance for the virus to mutate there and become resistant. These results have been published in two papers by Jendis et al 1996, 1998 (AIDS Research and Human Retroviruses 12, 1161, 1996 and AIDS Research and Human Retroviruses 14 (11), 999, 1998).

The strategies of managing retroviral infections known from the state of the art have in common that they are intended for the use in infected cells. Consequently, these strategies are not suitable for the prevention of retroviral infection. To defeat further spreading of retroviral infections there is a strong need for methods and means that are capable of stopping for example HIV transmission before a cell can be entered by the viral particles.

Coming from this state of the art, it is an object of the present invention to provide an alternative method and means for the specific inactivation of retroviruses.

Furthermore it is an object of the present invention to provide a pharmaceutical composition usable as medicinal product corresponding to said alternative method suitable for the inactivation of retroviruses.

### SUMMARY OF THE INVENTION

In the following we would like to give some more specific definitions of terms used within the context of the present invention:
In connection with the present invention a "cell-free environment" basically means administration "outside a cell". This means that a method or a pharmaceutical composition according to the present invention is used outside a cell, whereas the use in a vagina or inside a blood vessel is naturally comprised as this represents a body cavity formed of cells, but not the use within a cell. Consequently, also the use within the bloodstream or other body fluids of a human is to be understood as "outside the cell".

The term "anti-viral" relates to all types of virus defeating measures in connection with viruses that have an RNA or DNA genome replicating through an RNA or DNA intermediate stage. Consequently retroviruses like the Human Immunodeficiency Virus (HIV) are as well comprised as viruses like the Hepatitis B Virus (HBV) as this is a DNA virus replicating through a RNA intermediate stage and being designated due to this as pseudo-retrovirus. Most important in connection with the present invention is on one hand the concerted action of the enzymes ReverseTranscriptase (RT) and RNaseH and on the other hand the presence of a poly(A)-tail as part of a RNA. The disclosure is not restricted to retroviruses like HIV, but also to all know retroviruses like HIV-I and HIV-II as well as HTLV-I and HTLV-II (human T-cell leukemia virus type 1 and type 2) which could cause infections for humans and animals through RNA, RNA-DNA or a DNA intermediate stage.

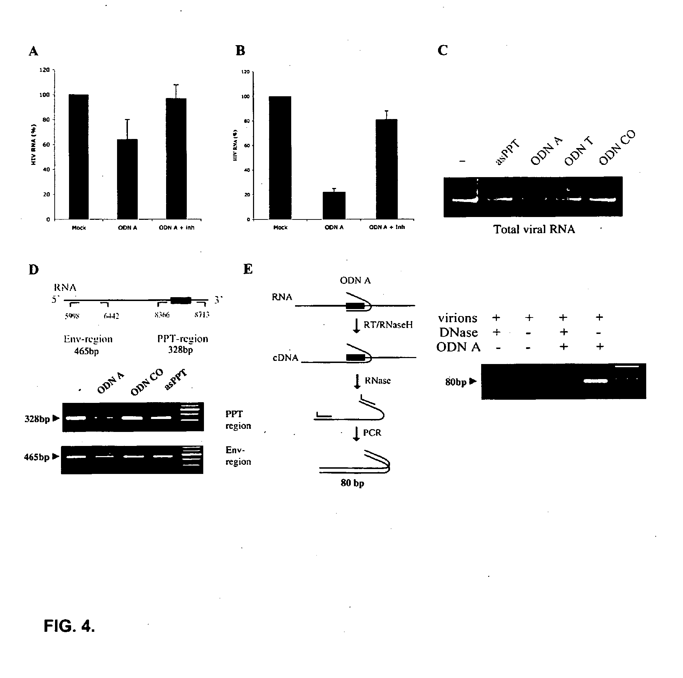

The term "oligonucleotides" (ON) summarises ODN (oligodeoxynucleotides) as well as ORN (oligoribonucteotides) or both ODN-ORN (chimera), ONs are 8 to 80 nucleotides in length. The ONs can be single-stranded (antisense) or double-stranded, whereby the double-stranded ON can be partially or fully self-complimentary. The ONs are targeted to a viral sequence and are either fully or partially complementary to the target sequence (some of which are listed below). While oligodeoxynucleotides (referred to as ODN) and oligoribonucleotides (referred to as ORN) or chimeras (ODN-ORN) or combinations thereof may be utilized with a preferred length of 8 to 80 nucleotides it is also intended to use mononucleotides for oligonucleotide synthesis or primer that will be elongated both by RT leading to activation of RNaseH and increasing the destruction of viral RNA (as shown in Fig. 4 E).

The "target sequence" can be the viral RNA genome. The target sequence is a polypurine tract, the 3-PPT of HIV-1.

Body fluids will be understood by those skilled in the art as blood, serum, plasma, saliva, tears, sperm, vaginal secretion or any other secret of a body.

The objects of the invention are solved by the features of the independent claims.

In general, the invention can be described as follows: In contrast to known mechanisms and treatments for inactivation of virus functionality the present invention is based on a completely different understanding of the interaction of synthetic oligonucleotides with viral nucleic acids. While so far interaction was directed to cell associated destruction of viral nucleic acid the present invention is based on the surprising effect that viral nucleic acid can be also affected outside a cell. An oligodeoxynucleotide (ODN) is able to affect the viral RNA also outside of the body or outside of a cell, as it is active inside the virion.

The antiviral action is due to the activation of a viral enzyme, which is present in the virus particle, the Reverse Transcriptase RT/RNaseH. The ODN targeted to the PPT is recognized by the RT/RNase H inside the virus and activates its activities. Thereby the viral RNase H cleaves the viral RNA prematurely, before the RNA is copied into DNA. This irreversibly leads to loss of the genomic information and failure to make a full-length DNA copy for a DNA provirus formation. The effect can be described as a viral suicide, because the virus itself causes the killing of its genome. The RT/RNase H activities are activated prematurely by the ODN. This corresponds to a correct step in viral replication, however prematurely. Mononucleotides activate the RT/RNaseH to generate new hybrids and enhance the destructive effect.

The effect is totally unexpected, because cellular enzymes were considered to perform the destruction of the viral RNA inside the cell, not outside of the cell in the virus particle (and only after DNA synthesis).

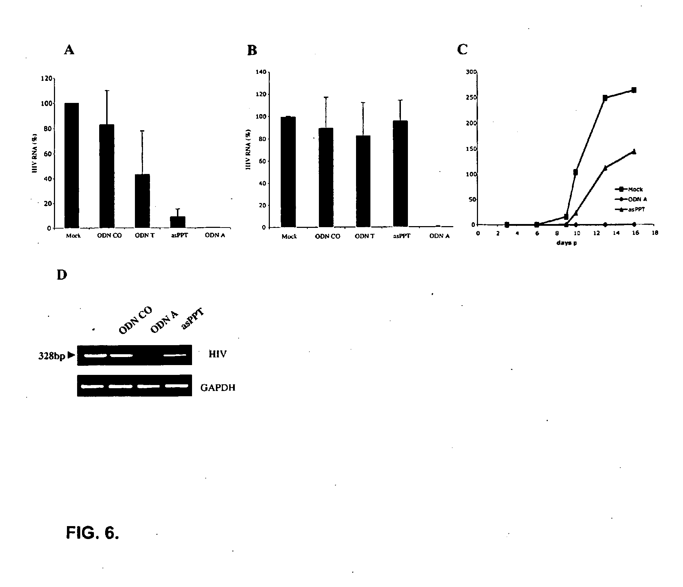

Thus, treatment of viral particles can abrogate the viral infectivity. Treatment of virus (HIV) in vitro for 4 hours and subsequent infection of a host cell for test of infectivity demonstrates that the virus has become inactive. The RNA is destroyed and no virus protein (p24) provided (Fig. 6). A dose dependence and kinetic analysis have been performed (Fig. 6).

The sequences as shown below are disclosed:

**Table 1: used Sequences (DNA or RNA or DNA/RNA)**

| SeQ.ID No. | kind/ name Sequence (direction: 5' to 3') | reference |
|---|---|---|
| 1 | HIV-1 PPT DNA | |
| | AAAAGAAAAGGGGGGACTGGAAGGG | |
| 2 | DNA | |
| | TTTTGTTTTGGGGGGTGTGGTTGGG | |
| 3 | ODN A 3' part,PPT (asPPT) | Fig. 1 A |
| | CCCTTCCAGTCCCCCCTTTTCTTTT | |
| 4 | ODN A 5' part, PPT | Fig. 1 A |
| | TTTTCTTTTGGGGGGTTTGGTTGGG | |
| 5 | DNA, ODN A | Fig. 1 B |
| | TTTTCTTTTGGGGGGTTTGGTTGGGTTTTCCCTTCCAGTCCCCCCTTTTCTTTT | |
| 6 | DNA, ODN D | Fig. 1 B |
| | TTTTCTTTTGGGGGGTCTGGTTGGGTTTTCCCTTCCAGTCCCCCCTTTTCTTTT | |
| 7 | DNA ODN A(C-T) | |
| | TTTTTTTTTGGGGGGTTTGGTTGGGTTTTCCCTTCCAGTCCCCCCTTTTCTTTT | |
| 8 | ODN A(C-G) | |
| | TTTTGTTTTGGGGGGTTTGGTTGGGTTTTCCCTTCCAGTCCCCCCTTTTCTTTT | |
| 9 | ODN B 3' part | Fig. 1 B |
| | CCCTTCCAGTCCCCCCTTTTCTTT | |
| 10 | ODN B 5' part | Fig. 1 B |
| | TTTCTTTTGGGGGGTTTGGTTGGG | |
| 11 | DNA, ODN B | Fig. 1 B |
| | TTTCTTTTGGGGGGTTTGGTTGGGTTTTCCCTTCCAGTCCCCCCTTTTCTTT | |
| 12 | asPPT | Fig. 1 B |
| | CCCTTCCAGTCCCCCCTTTTCTTTT | |
| 13 | ODN SC | Fig. 1 B |
| | TTTGGGGGGTTCTTCCTCCTTTCCTTTTTCGCCCGTCCGTTGCGTTGATTTTTT | |
| 14 | ODN T | Fig. 1 B |
| | AAAAGAAAAGGGGGGACTGGTTGGGTTTTCCCTTCCAGTCCCCCCTTTTCTTTT | |
| 15 | ODN H | Fig. 1 B |
| | TTTTCTTTTGGGGGGACTGGTTGGGTTTTCCCTTCCAGTCCCCCCTTTTCTTTT | |
| 16 | ODN NT | Fig. 1 B |
| | TTTTCTTTTGCGCGCTTTGGTTGGGTTTTCCCTTCCAGTCCCCCCTTTTCTTTT | |
| 17 | ODN D | Fig. 1 B |
| | TTTTCTTTTGGGGGGTCTGGTTGGGTTTTCCCTTCCAGTCCCCCCTTTTCTTTT | |
| 18 | ODN CG | Fig. 7 a |
| | TCTTTTTGCGCGCTTTGTTTGTTTTTTCTTTCATTCCCCCCTTTTTCT | |
| 19 | asEXT | Fig. 1 B |
| | CATGCACGTGTGACGTT | |
| 20 | ODN M | Fig. 7a |
| | TCTTTTTGGGGGGTTTGTTTGTTTTTTCTTTCATTCCCCCCTTTTTCT | |
| 21 | ODN F | Fig. 7a |
| | AGAAAAAGGGGGGAATGAAAGATTTTTCTTTCATTCCCCCCTTTTTCT | |
| 22 | ODN S | Fig. 7a |
| | TCTTTTTGGGGGGAATGTTTGTTTTTTCTTTCATTCCCCCCTTTTTCT | |
| 23 | ODN M(AS) | Fig. 7 a |
| | TCTTTCATTCCCCCCTTTTTCT | |
| | ODN/ORN chimaeras | |
| 24 | cccuuCCAGTCcccccuuuucuuuu | |
| 25 | cccuuccAGTCCccccuuuucuuuu | |
| 26 | CCCTTCCaguccCCCCTTTTCTTTT | |
| | (small letters ORN, capital letters ODN) | |
| | siRNA5'part (above) and siRNA3'part (below) | |
| 27 | AGAAAAGGGGGGACUGGAATT | |
| 28 | UUCCAGUCCCCCCUUUUCUTT | |

Seq.ID 3 and Seq.ID 4 are linked by a T4-spacer (TTTT) to the so-called ODN A (Seq.ID 5).

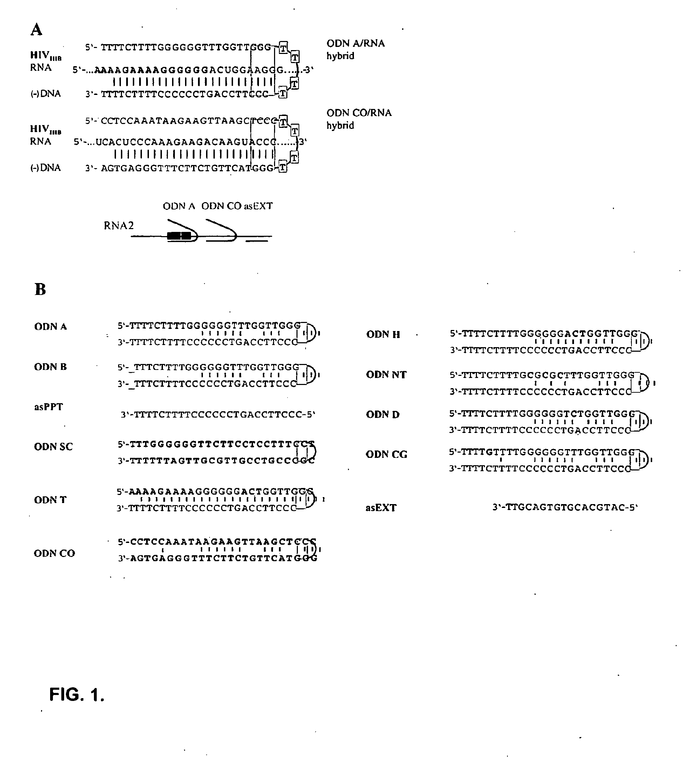

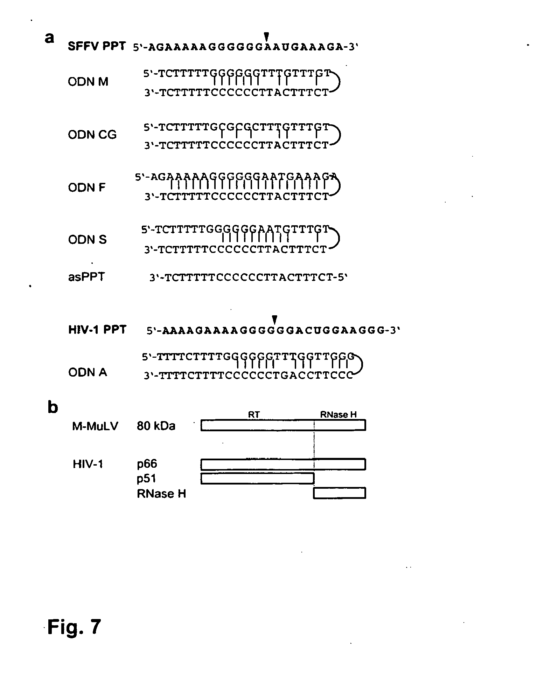

It should be noted that according to the figures, in particular figure 1 and 7 the sequences as shown (upper partial sequence strand and lower partial sequence strand) are connected via a T4-linker (four thymidines), indicated as a curved line, which is shown in detail in Fig. 1A as an example. It should be further noted, that, however, in general the T4 linker can be replaced by any other linker able to stabilize the two strands.

It is further within the scope of invention that also "upper" and "lower" sequence strands of ODNA in Fig. 1 and 7 regardless of whether the strands are connected by a T4 linker or not are an object of the invention (3' part and 5' part, as shown i.e. for ODN A and ODN B). Therefore, if within the description of this invention is referred to one or more of the sequences of Seq.ID NO. 5, it should be noted that also these parts / strands of said sequences are within the scope of invention.

Table 2 shows target regions comprising conserved binding regions for oligonucleotides / oligoribonucleotides.

**Table 2: Target sequences**

| Seq.ID | Sequence | kind / name |
|---|---|---|
| 29 | AAAAGAAAAGGGGGGACUGGAAGGG | 3-PPT of HIV-IIIB RNA |
| 30 | AAAAGAAAAGGGGGGAUUGGGGGG | 5-PPT of HIV-IIIB RNA (internal PPT) |
| 31 | AGUGGCGCCCGAACAGGGACUGAAAGCGAAAGGGAAA | RNA, Primer-binding site (PBS) of HIV |
| 32 | AAAAAAAAAAAAAAAAAAAAAAAAAAAAAAAA | RNA, Poly(A)-tail |
| 33 | UUUAAGAAAAAGGGGGGACUGGAAGGGCUUAA | RNA, PPT OF HIV Bal |
| 34 | AGAAAAAGGGGGGAAUGAAAGA | RNA, PPT OF SFFV |
| 35 | AAAAGAAAAGGGGGGACUGGAAGGG | 3-PPT of HIV variants |
| 36 | AAGAGAAAAGGGGGGACUGGAAGGG | 3-PPT of HIV variants |
| 37 | AAAAGAAAAGGGGGGACUGGAUGGG | 3-PPT of HIV variants |
| 38 | AAAAGAAGAGGGGGGACUGGAAGGG | 3-PPT of HIV variants |
| 39 | AAAAGAACAGGGGGGACUGGAAGGG | 3-PPT of HIV variants |
| 40 | UAAGAAAAAGGGGGGACUGGAAGGG | 3-PPT of HIV variants |
| 41 | AAAGACUGGGAGGAGUUGGGGGAGGAGA | Hepatitis B Virus RNA |
| 42 | GAGGCGAGGGGCGGCGACUGGUGAGACGCCAAAA | PSI HIV RNA, Ref. (1) |
| 43 | CCGUGUUGGGGGCUUUGGGGCAUGGACA | Epsilon HBV RNA, Ref. (2) |

| | | |
|---|---|---|
| Ref. (1): Amarasinghe G.K. et al., J. Mol. Biol., 301, 491 (2000) Ref. (2): Pollack J.R. and Ganem D., J. Virol.,67, 3254 (1993) | | |

An important improvement for the fight against HIV could be the reduction of transmission during sexual intercourse. This is a recent very important goal, since there may not be a vaccine. Furthermore other virus infections in the vagina increase the risk for transmission of HIV. Even circumcision is considered as a hygiene measure to reduce the rate of transmission, which would be a factor of 2. However, the use of the present invention is a microbicide against HIV-1 infections through sexual transmission.

Meanwhile microbicide against Herpes Simplex Virus, HSV, infection of the vagina which is a serious cofactor for the HIV infection, has been described by (Palliser et al., Nature 439, 2006, 89-94). The drug is intended for administering into the vagina for antiviral action so that the HSV will not be able to infect. A decrease of HSV infections would strongly decrease the chance for a HIV infection. The formulation of the drug has to avoid any mucosal irritation or lesions in order not to give rise to the opposite effect, such as a better infection instead of prevention.

In the examples below the invention is explained in more detail. To demonstrate the efficiency of the viral inactivation according to the invention, we used a model virus replacing HIV in a small animal, called SFFV (Spleen Focus Forming Virus), because there is no mouse model for HIV. It is a virus similar to HIV, however causing cancer in the spleens. Below we describe the surprising effect that SFFV virus titer in the blood of fully infected mice can be reduced by a single dose of ODN applied to the tail vein, by about 10fold, depending on the dose. The effect is due to destruction of the virus by the ODN in the blood circulation. This is inside the body, but not inside a cell, especially a viral infected cell.

The reduction of the virus load within the blood stream is completely surprising as the effect of applying oligodeoxynucleotide (ODN) or oligoribonucleotide (ORN) or ODN/ORN chimera or a combination thereof is demonstrably after 4 hours. The so far known effects in a cellular context become detectable after at least 12 hours. This means that the antiviral treatment of the present invention is much more effective than the ones known from the state of the art with the additional advantage that the subject matter of the present invention allows prevention of viral infections and is not restricted to the treatment of already viral infected cells.

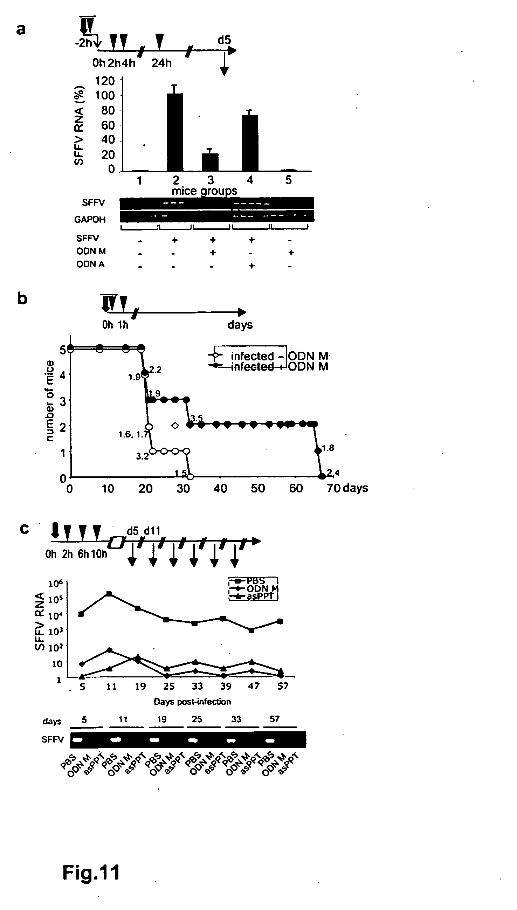

Next we demonstrate below that treatment of the virus at the time of infection can abrogate viral infectivity as measured by a twofold increased survival time of 2 out of 5 mice by twofold (Fig. 11 B). This shows an antiviral effect during uptake of the virus by the cells or the body.

Further, we demonstrate that pretreatment of the virus with the ODN or antisense (asPPT) in a small test tube (the equivalent of a vagina) for 1 to 4 hours and subsequent infection of the mouse can completely abrogate viral infectivity in the body in a dose dependent manner.

This is shown in the Figure 11. There is no virus detectable and the mice remain healthy for 30 days. This corresponds to protection of a female from virus infection during sexual intercourse.

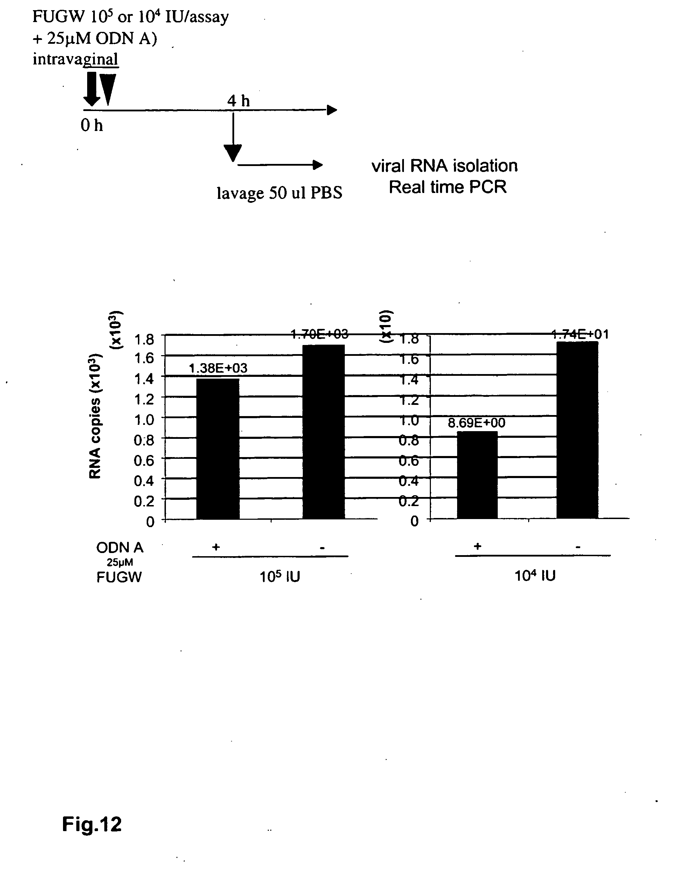

Next we performed an intravaginal treatment with ODNA A of a recombinant retrovirus and recovered the virus after 4h by lavage. The titer was reduced by a factor of two (Fig. 12).

In summary of the above, the surprising results are:
Virus particles can be inactivated by their treatment according to the present invention, whereby
   - Inactivation can occur in the blood stream,
   - Inactivation can occur during infection by simultaneous treatment with the ODN,
   - Inactivation can occur *ex vivo* in a vessel before uptake of the virus, mimicking a vagina,
   - Inactivation can occur in the vagina.

From the above it should be understood that according to the invention the use of an oligodeoxynucleotide or capable of binding at least in part to the extended PPT target regions of HIV-1 viral RNA for the inactivation of viral infectivity outside in a cell-free environment is intended, as claimed.

To prevent degradation of the used nucleotide chain it is - if necessary - intended that the backbone of the nucleotide chain is stabilized by secondary modifications; preferably the modification comprises a modification of the nucleotide backbone from phosphodiesters to phosphorothioates.

According to the invention sequences Seq.ID NO. 5 is especially preferred as ODN..

In the presence of monodeoxynucleotides the RT/RNaseH can be activated for cDNA synthesis, e.g. by primer extension at the ODN A and thus increase the length of the hybrid region (Fig. 4E). This would allow the RNaseH to further destroy more viral RNA due to the concerted action of the RT/RNaseH. Thus, more ODN is generated and more viral RNA is destroyed.

The pharmaceutical agent for use according to the invention should be useful for prevention of viral infections after contact with virus containing fluids and/or the reduction of virus load with viruses replicating through a RNA intermediate stage. A medicinal product like a microbicide should therefore be capable to prevent retroviral infections even if there is direct contact with retroviral contaminated liquids, e.g. at mucosal surfaces.

As already mentioned above, mononucleotides, especially monodeoxynucleotides activate the RT/RNaseH to generate new hybrids and enhance the destructive effect.

It is also intended according to the present invention, to use the pharmaceutical preparation as described above in combination with one or more supplementary agents from the group of antiviral, fungicidal or antibacterial agents, anti-cancer agents and/or immunostimulators or immunomodulators. The pharmaceutical agent is prepared for the application on the vaginal mucosa. The method according to the invention is intended for the use on vaginal mucosal surfaces. Consequently it will be understood by those skilled in the art that a pharmaceutical preparation according to the invention comprises any preparation that is suitable for applying a pharmaceutical agent for use according to the invention.. This means especially forms of administration such as suppositories, lotions, creams, ointments, sprays or liquids, also used in combination with a condom as lubricant.

A pharmaceutical agent for use according to the invention is intended for the prevention of HIV-1 viral infection during sexual intercourse. Although, multi drug resistant HIV patients could benefit from the invention. It is further disclosed to prevent mother to child transmission during birth delivery by applying the pharmaceutical composition to the mother shortly before birth delivery, Further, the disclosure is of importance for any kind of surgery (also oral and dental surgery) in respect of prevention virus transmission during surgery by reduction of virus load in body fluids (i.e. blood, saliva).

The invention will be described by examples and figures below, where both are meant to illustrate the invention and not to limit it in any way.

### BRIEF DESCRIPTION OF THE DRAWINGS

- **Fig. 1**: Sequences of ODNs
- **Fig. 2**: ODNs induce the RT/RNaseH-dependent cleavage of the viral RNA in sequence-specific manner
- **Fig. 3**: Comparative efficiency of ODNs in virions
- **Fig. 4**: ODN A mediates RT/RNaseH-dependent degradation of viral RNA
- **Fig. 5**: Analysis of infectivity of ODN A-treated virions
- **Fig. 6**: ODN A abrogates infectivity of HIV virions
- **Fig. 7**: Oligonucleotides and domains of Reverse Transcriptase / RNase H
- **Fig. 8**: ODN mediated specific cleavage within PPT
- **Fig. 9**: Effects of ODNs on SFFV virions and SFFV-infected cells
- **Fig. 10**: ODN M effects analyzed in the SFFV mouse model
- **Fig. 11**: Abrogation of SFFV infectivity by early treatment with ODN M
- **Fig. 12**: intravaginal treatment (mice) of virus with ODN and a subsequent test for inactivation of the virus

### DESCRIPTION OF THE INVENTION AND EXAMPLES

### In vitro analysis of ODNs

To demonstrate the effects of an oligodeoxynucleotide according to the invention, oligodeoxynucleotide A (ODN A), which consists of a 25mer antisense, and a 25mer passenger strand, connected by four thymidines (T4) was used. The sequences of the strands are partially complementary. The ODN A was phosphorothioated at each end (3 bases) and in the T4 linker. ODN A was targeted to the extended PPT of HIV-1. We also used a few structural variants of ODN A: ODN T, which has a passenger strand fully complementary to the antisense strand; the single-stranded antisense PPT (asPPT) which lacks the passenger strand and the linker; ODN H and ODN NT, which both have three nucleotide changes at different positions of the passenger strand; ODN CG and ODN D, both having a single nucleotide change at different positions of the passenger strand; and ODN B which has one nucleotide removed from both 5' and 3' ends. ODN Sc has the same length and nucleotide composition as ODN A but a randomized sequence of both strands. ODN CO has a secondary structure similar to ODN A but targets sequences downstream of the extended PPT. Antisense oligodeoxynucleotide asEXT, which targets sites outside of the extended PPT, was also used (Fig. 1).

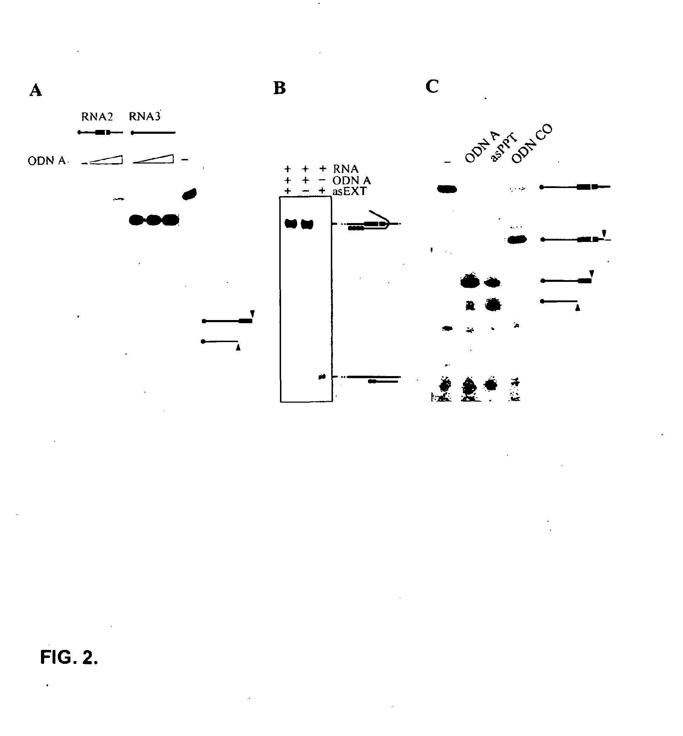

We previously showed that the ODN A at high concentrations (1 µM or more) inhibits the RT/RNaseH activity in vitro.¹² To check the effect of ODN A at lower concentrations of RT/RNaseH-dependent cleavage of RNA, we tested various concentrations of ODNs in an RNase H cleavage assay. We used synthetic 5'-end-labelled RNA2, which contains the extended PPT and sequences of the viral genome close to the PPT. As can be seen in Figure 2A RNA2 was indeed cleaved with 10 nM ODN A, but not with 1µM ODN A suggesting that at low concentration ODN A can mediate the cleavage of the RNA by RT/RNaseH. The cleavage depended on the presence of the PPT on the RNA, since RNA3, which does not harbour the PPT sequence, was not affected by ODN A-mediated cleavage (Fig. 2A). For the present study we used low concentrations of the ODNs.

To test whether an ODN-mediated activity would preferentially occur at the PPT, we analysed ODNs targeted to the extended PPT (ODN A) or to a region outside of the PPT (ODN CO and an external antisense oligodeoxynucleotide asEXT) in vitro. We used RNA2, which contains the PPT and the binding sites for ODN A, asEXT and ODN CO. As shown in Figure 2B, RT/RNaseH initiated primer extension of the ODN A more efficiently than the antisense primer outside of the PPT (asEXT), demonstrating a preference for recognition of the PPT region. Indeed, in the reaction mixture containing both ODN A and asEXT primers, extension products, predominantly synthesized by the RT/RNaseH, started with ODN A and not asEXT (Fig. 2B). Furthermore, an RNase H cleavage assay revealed more efficient cleavage of RNA2 induced by ODN A compared to the control ODN CO, which has a similar secondary structure as ODN A but is targeted to a site outside of the PPT (Fig. 2C, lanes ODN A and ODN CO). Thus, the ability of the ODN A to mediate RT/RNaseH-dependent cleavage of the viral RNA is PPT-dependent. Importantly, the effect of ODN A was slightly stronger compared to that of single-stranded antisense oligonucleotide asPPT, suggesting that the passenger strand of the ODN A is important for more efficient viral RNA cleavage (Fig. 2C, lanes ODN A and asPPT).

### ODN-mediated cleavage of RNA in HIV virions

HIV replication is a complex process which includes reverse transcriptase processivity, RNase H activity for hydrolysis of the viral RNA, and requires the presence of the viral nucleocapsid protein and an appropriate secondary structure of the RNA.¹⁴⁻¹⁶ The optimization of all of these conditions may not be fully met in in vitro assays, however more physiological conditions are encountered in HIV virions. Additionally, it has been shown, that phosphorothioated oligodeoxynucleotides can be internalized in cells without any delivery systems.^{12,17} Therefore, in order to simulate the situation in vivo, we incubated intact virions with ODNs in cell culture medium without any detergents or ODN-carriers. We used a higher concentration of ODNs than in in vitro studies, since the cellular uptake of oligonucleotides is low and the majority of ODNs may not penetrate through the cellular membrane. We assumed that in virions similar to cells only a small portion of ODNs could be internalized.

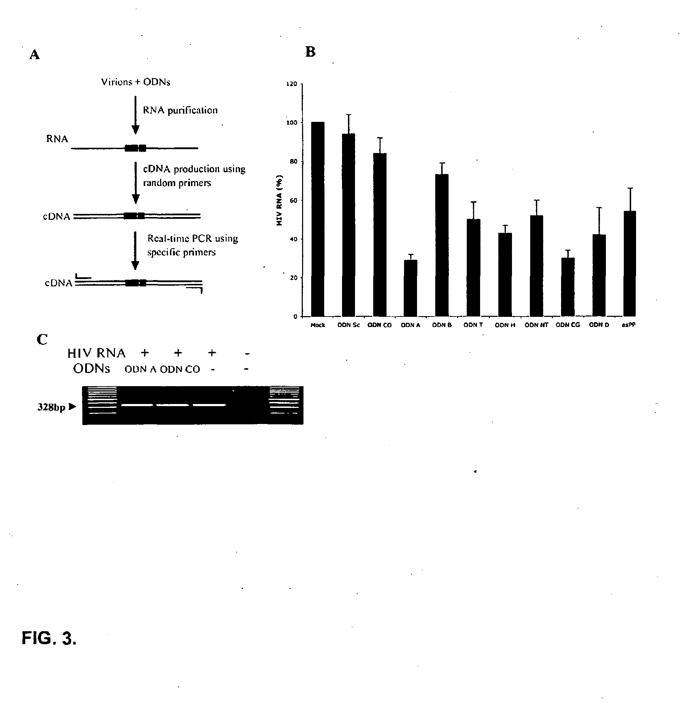

In our study we used ODN A, ODN CO, asPPT and additional control oligodeoxynucleotides described in Figure 1B: ODN T, ODN B, ODN NT, ODN and ODN CG. HIV virions were incubated with ODNs, viral RNA was then purified, reverse transcribed and the amount of undigested RNA was quantified by real-time PCR using a set of primers covering the PPT region of the HIV genome as indicated in Figure 3A. We observed profound difference in the antiviral activities between various structural variants of the ODNs. We could detect a significant decrease in the amount of amplification product in the case of ODN A compared to other control ODNs. Only ODN CG showed an antiviral activity similar to ODN A. All other variants of ODN A showed less efficient cleavage of the viral RNA. These variants include deletions (ODN B), the scrambled nucleotide sequence (ODN Sc), additional hydrogen bonds between antisense and passenger strands (ODN T and ODN H), alteration of polyG/polyC duplex (ODN NT), or removal of the passenger strand (asPPT) (Fig. 3B). Thus, the length and the sequences of both strands of the ODN proved to be important for the antiviral effect.

To test whether ODNs may interfere with the RT-PCR assay, used as a readout of RNA degradation, we purified the viral RNA pre-incubated with ODNs under conditions facilitating hybridization. Hybrid complexes were then re-purified and RT-PCR analysis was performed as described in Materials and methods. As shown in Figure 3C binding of ODNs to RNA did not alter the accumulation of the amplification products thus excluding the possibility that ODNs may interfere with the RT-PCR.

In order to prove that the reduction of viral RNA levels was indeed due to the enzymatic activity of the RNase H domain of the RT/RNaseH, we used Illimaquinone, previously described to specifically inhibit the RNase H activity of HIV RT/RNaseH. We permeabilized virions with 0.1% NP-40 to facilitate the uptake of Illimaquinone and measured the viral RNA degradation by real-time PCR. As shown in Figure 4A, in the presence of Illimaquinone the RT/RNaseH failed to induce cleavage and the level of RNA remained as in the uncleaved control. Similar results were observed when the reaction mixture was supplemented with additional recombinant RT/RNaseH to intensify the viral RNA degradation (Fig. 4B). To test the integrity of HIV RNA, we then analyzed the total viral RNA extracted from virions pre-incubated with ODNs by agarose gel electrophoresis in the absence of Illimaquinone. As shown in Figure 4C, a significant reduction of the viral RNA was observed when virions were incubated with ODN A in comparison to control ODNs. In order to learn more about the RNA degradation, we performed RT-PCR using primers covering either the PPT region or primers covering a segment of the HIV genome 1924 nucleotides upstream of the PPT (Env-region). In both cases a significant reduction in the amount of amplification products was observed in the case of ODN A compared to other ODNs (Fig. 4D).

It has been previously shown that some reverse transcription can take place within extracellular HIV virions. Therefore, it is reasonable to assume that following ODN A-mediated cleavage by the RNase H activity of RT/RNaseH, the polymerase activity extends the ODN A as an artificial primer and the RNase H activity degrades the viral RNA in the concerted action. Indeed, incubation of virions with ODN A led to cDNA production starting with ODN A as registered by PCR using ODN A- and viral cDNA -specific primers (Fig. 4E).

### Pre-incubation of HIV virions suppresses the infectivity of viral particles in cell culture

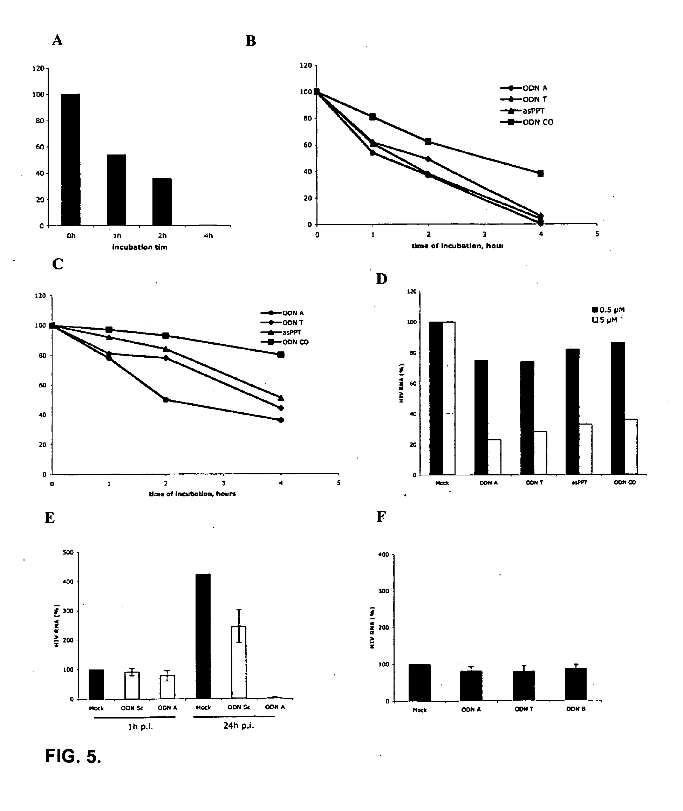

So far we have demonstrated ODN A-mediated HIV RNA degradation in intact virions. As a next step we investigated the antiviral effect of ODNs by testing the infectivity of treated virions in cell culture studies. We used ODN A, ODN T, as PPT and ODN CO. 2 x 10⁵ C81-66/45 cells were infected with virions pre-incubated with different ODNs at 0.01 MOI for 1, 2 or 4 hours prior to infection. RNA was extracted from infected cells and HIV replication was analysed by real-time PCR 3 days post-infection. The time course analysis revealed that incubation of virions with ODN A for 4 hours was sufficient for complete suppression of viral replication in infected cells (Fig. 5A), ODN CO was clearly less efficient, while ODN T and asPPT showed low but detectable levels in contrast to ODN A (Fig. 5B). Importantly, reduced viral replication in infected cells correlated with the kinetics of degradation of viral RNA within virions (Fig. 5C), attributing the antiviral effects of ODNs predominantly to virion-associated enzymes. The antiviral effect of ODN A appears to be dose-dependent, since the infection of C88-61/45 cells with ODN-pre-treated virions at 100-fold higher MOI (MOI of 1) resulted in only partial suppression of HIV replication. However, the viral replication could be reduced again by increase of the ODN concentration, yet with reduced sequence-specificity (Fig. 5D). This result demonstrates the concentration-dependence of antiviral effect of ODN A.

Pre-incubation of virions with ODN A for 4 hours and infection at low MOI (MOI of 0.1) did not significantly interfere with binding of virions to the host cells. Indeed, analysis of HIV RNA in infected cells 1 hour post-infection showed that virions, pretreated with ODN A for 4 hours, were taken up by cells only 14% less efficiently compared to virions treated with ODN Sc (Fig. 5E). However, viral RNA was completely degraded only in the presence of ODN A, as can be seen 24 hours after infection (Fig. 5E). Importantly, virions pre-treated with ODN B or ODN T showed uptake similar to ODN A, as demonstrated by analysis of HIV RNA in infected cells 1 hour post-infection (Fig. 5F). Thus, ODN A-mediated abrogation of infectivity of pretreated virions may not exclusively depend on the degradation of RNA inside of the virions. The ODNs, including ODN A, may also bind to virions and mediate the cleavage of the viral RNA inside of infected cells, suggesting that intracellular factors could also play a role in this process.

To test the specificity of suppression of viral replication, the effect of ODN A on the infectivity of virions was compared with control ODNs. Virions were pre-treated with the ODNs and HIV replication in infected cells was analysed by real-time PCR, 5 days (Fig. 6A) or 20 days post-infection (Fig. 6B) and by measuring p24 production in the supernatant of the infected cells (Fig. 6C). These results were also confirmed by RT-PCR, 11 days p.i (Fig. 6D), and by visual inspection of syncytia formation (Fig. 6E). Strikingly, cells which received virions pre-treated with ODN A, showed no sign of virus replication for at least 20 days after infection. Treatment of the virions with control ODNs also led to a suppression of HIV replication, but the effect was transient and 20 days post-infection the virus was able to propagate again and reach the level of the untreated control, which was not the case with ODN A.

### SFFV as retroviral mouse model

There is no simple HIV mouse model available for replicating HIV. We therefore used the oncogenic replication-defective SFFV, pseudotyped by the replication-competent MuLV helper virus as model virus in mice. SFFV causes spleen foci through its short envelope protein gp55, which activates the Erythropoietin receptor in the absence of the hormone and stimulates cell proliferation. MuLV resembles SFFV in sequence except for an extended gp80 envelope protein. SFFV has a typical retroviral PPT, slightly shorter than that of HIV (Fig. 7a). The polymerase (pol) gene of MuLV codes for an 80 kDa RT/RNase H (p80), which can form homodimers but is active mainly as monomer (Fig. 7b), whereas the HIV RT/RNase H is a heterodimer of p66 and p51 with and without RNase H.

SFFV replicates to high titers in mice, whereby the spleen is a virus-shedding reservoir. The virus stock is recovered from spleen homogenates after full-blown disease, at about day 20 post infection (pi). The clarified supernatant contains around 2x10⁵ focus forming units (FFU) per ml. Infection was normally performed by intravenous (iv) injection of 4x10³ SFFV. After 5 days the blood was recovered (100 microliters) for detection of the viral RNA. The equivalent of 0.05 microliters was enough for RNA detection by 40 cycles. One FFU corresponds to about 6x10⁴ RNA copies or 3x10⁴ virus particles.

### In vitro analysis of ODNs

In this study we used an oligodeoxynucleotide M (ODN M), targeted to the extended murine viral PPT of SFFV, consisting of a 22-mer antisense, and a 22-mer second strand, connected by four thymidines (T4). The sequences of the strands are partially self-complementary, which may allow formation of a hairpin-loop structure (Fig. 7a). The ODN M was phosphorothioated at 3 bases at each end and in the T4 linker. We also used a few structural variants of ODN M: ODN F, which has a second strand fully complementary to the antisense strand; the single-stranded antisense PPT (asPPT), which lacks the second strand and the linker; ODN S, which has two nucleotide changes in the second strand; ODN CG having a triple nucleotide change in the second strand; and the HIV-PPT-specific ODN A which has multiple changes in both antisense and second strand for the SFFV target RNA, while its antisense strand is fully complementary to the HIV-1 PPT RNA (Fig. 7a). ODN A has been characterized previously with HIV.

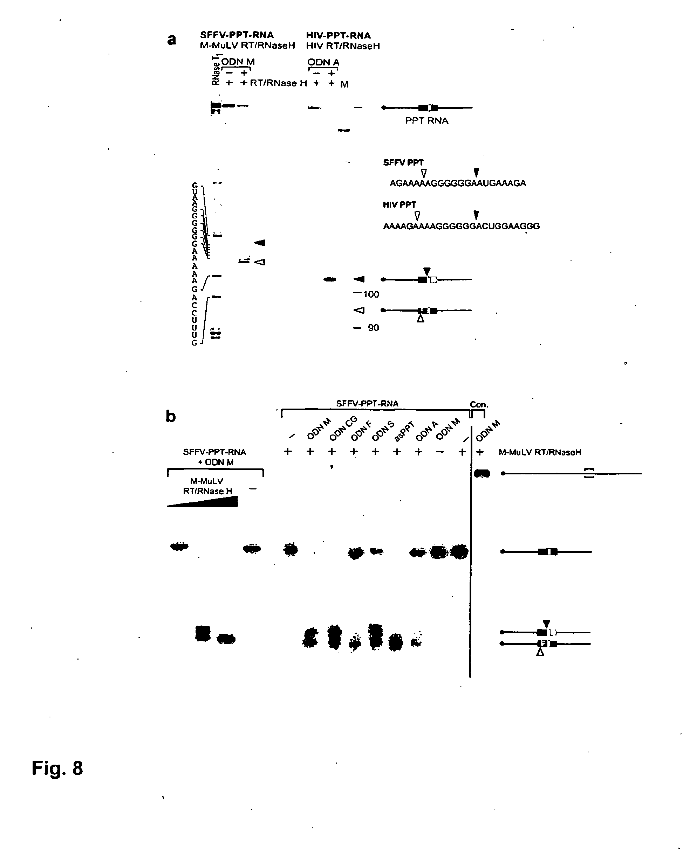

To test, whether ODN M would have similar effects as ODN A, we tested the ability of ODN M to induce cleavage of SFFV RNA in the presence of the recombinant M-MuLV RT/RNase H. We used synthetic 5'-end-labelled RNA, which contains the extended PPT of SFFV and sequences of the viral genome close to the PPT. As can be seen in Fig. 8a the synthetic fragment of SFFV RNA was indeed cleaved in the presence of ODN M. The cleavage sites were similar to the ones induced by ODN A in HIV RNA (Fig. 8a). M-MuLV RT/RNase H induced ODN M-mediated cleavage in a dose-dependent manner (Fig. 8b left). The cleavage induced by the various ODNs leads to degradation of the SFFV PPT-RNA *in vitro* as shown (Fig. 8b right). The cleavage efficiency of the RNA by asPPT was similar to that of ODN M. The cleavage depended on the presence of the PPT on the RNA, since control RNA, which does not harbor the PPT sequence, was not affected by ODN-mediated cleavage (Fig. 8b right, Con.). These results from *in vitro* experiments suggested to us, that the mechanism of action of ODN M is similar to that of ODN A against HIV RNA described previously. We concluded that SFFV could serve as model for HIV.

### Effect of ODNs on virions

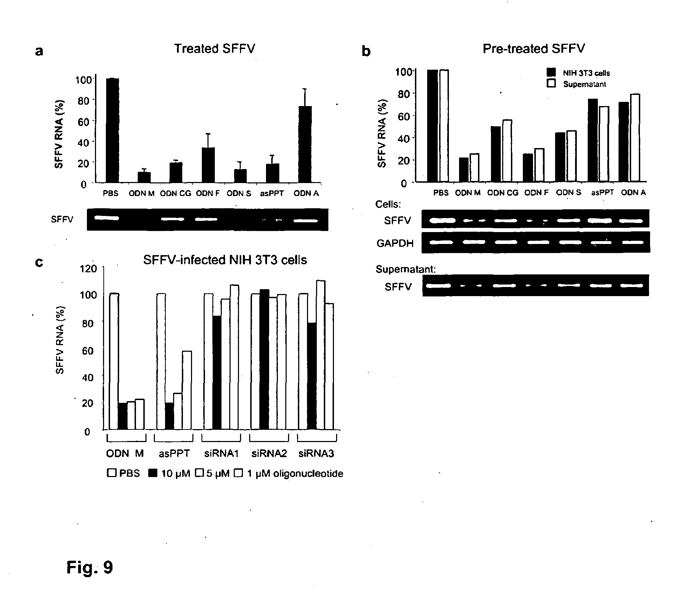

In order to further characterize antiviral effects of ODNs, we tested ODN M, asPPT and structural analogs of ODN M in SFFV virions, which contain endogenous RT/RNase H and have an appropriate secondary structure of the RNA. Permeabilized virions were incubated with ODNs, viral RNA was then purified, reverse transcribed and the amount of undigested RNA was quantified by real-time PCR using a set of primers harboring the PPT region of the SFFV genome. The result shows the amount of intact RNA in % as bars and amplified PCR products on gels below (Fig. 9a). We observed differences in the antiviral activities between various ODNs. ODN M showed the strongest effect compared to control ODNs or the single-stranded asPPT DNA. Thus, the presence as well as the sequences of both strands of the ODN contributed to the antiviral effect. We noticed previously that the second arm of the ODN is more important intracellularly than *in vitro.*

As next step we investigated the antiviral effect of ODNs by testing pre-treated virions for residual infectivity as has been described before for HIV particles. SFFV particles were pretreated with ODN M or its variants and used to infect NIH 3T3 cells. After three days RNA was extracted from infected cells and from the supernatants and SFFV was quantified by real-time RT-PCR and analysis of amplification products (Fig. 9b). As can be seen, ODN M suppressed viral replication in infected cells as well as in the supernatant; ODN CG, S, A, and as PPT were clearly less efficient, while ODN F showed similar activity compared to ODN M.

In the next experiment NIH 3T3 cells were infected with SFFV for 2h and then treated with three different concentrations each of ODN M, asPPT, and three different siRNAs. The antiviral effect of ODN M is slightly superior to the asPPT but much stronger compared to siRNA under these conditions (Fig. 9c). siRNA applications are known to require carrier, which we do not need for application of the ODNs against the virus or HIV-infected cells. siRNAs have previously shown efficacy against newly infecting HIV, however only in the presence of carrier.

### In vivo analysis of ODNs in an SFFV mouse model

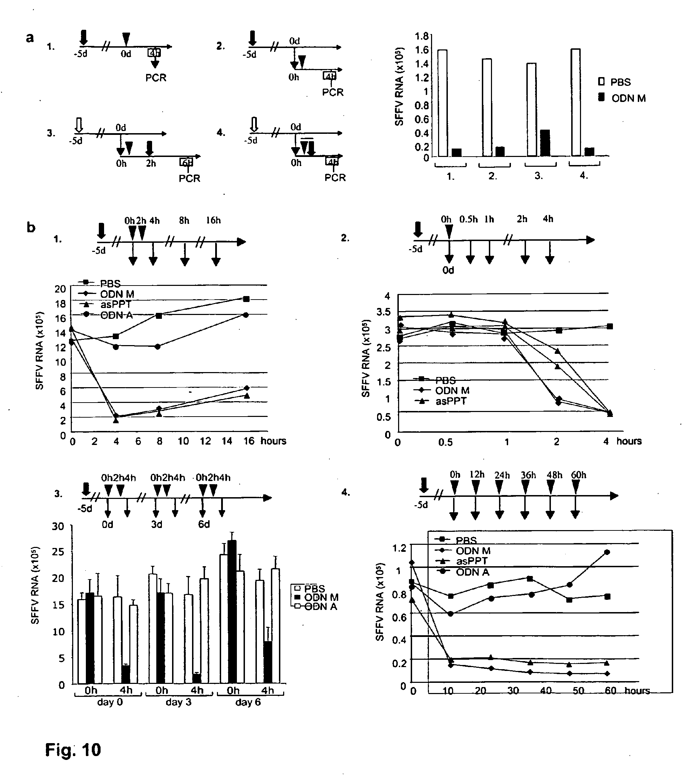

A number of experiments were carried out to evaluate the effect of ODNs on the SFFV mouse model. We treated 5-day-SFFV-infected mice (thick arrow) with ODN M (triangle) and measured the plasma RNA levels 4 hours after the *in vivo* therapy by real-time RT-PCR (Fig. 10a.1). In a second experiment we isolated the plasma of 5-day-SFFV-infected mice and treated it *ex vivo* with ODN M for 4 hours (Fig. 10a.2). In the third experiment we isolated uninfected plasma, incubated it *ex vivo* with ODN M for 2 hours followed by the addition of SFFV for 4 more hours (Fig. 10a.3). Finally we incubated ODN M and SFFV together in normal mouse plasma *ex vivo* for 4 hours (Fig. 10a.4). In all these experiments we were able to detect by PCR a similar reduction of SFFV RNA levels in the plasma indicating that ODN M directly acts on SFFV leading to destruction of the viral RNA.

To elucidate further the antiviral effect of ODN M *in vivo* we set up different therapeutic regimens. We infected mice with SFFV and then treated them 5 days later twice at an interval of two hours with either ODN M (1 mg), asPPT, ODN A as control, or PBS. The RNA was extracted 4, 8 and 16 hours later for PCR analysis (Fig. 10b.1). The viral RNA decreased approximately 6-fold in the plasma and started to slowly increase again 16 hours post-therapy.

In the next study, we used a single injection as treatment protocol and assessed the antiviral effect of both ODN M and asPPT in a short time-course. Plasma was taken from mice at 0.5, 1, 2, and 4 hours after the single treatment (Fig. 10b.2). A strong decrease of the number of SFFV RNA copies was detectable 4 hours after treatment. We noticed that 2 hours after beginning of the therapy, the effect of ODN M was stronger than that of asPPT suggesting that ODN M may follow a different mechanism than asPPT. We conclude from Fig. 10b.2 that a single injection is sufficient to decrease the number of RNA copies by approximately 6-fold 4 hours after the beginning of the therapy.

In order to test, whether the SFFV titer increases again after ODN M-treatment, and whether it remains sensitive for ODN M, we treated 5-day-SFFV-infected mice at 3 days intervals with a double dose within 2 hours intervals. Plasma was taken before and 4 hours after treatment and assessed for viral RNA levels (Fig. 10b.3). ODN M exerted a strong antiviral effect with a reduction of RNA copies of 6-fold at day 0 compared with the control. At day 3, the effect is even stronger with 8-fold decrease of the SFFV RNA level. As expected, ODN A showed no effect on viral replication, asPPT was not tested.

To obtain a more continuous effect of ODN M and asPPT on SFFV replication we treated the SFFV-infected mice every 12 hours (Fig. 10b.4). Indeed, this therapy showed a continuous effect throughout the period of treatment which is shown for up to 60 hours reflected by a plateau with a decrease of SFFV RNA levels by about 10-fold for ODN M and 5-fold for asPPT. ODN A showed no significant effect on the decrease of the RNA level after the therapy. The lack of response is in accordance with our previous *in vitro* studies on the sequence-specificity of the ODN A.

Taken together these results demonstrate that ODN M has a strong antiviral effect against SFFV detectable 4 hours after beginning of the therapy. Depending on the regimen of treatment, the antiviral efficiency can be transient or longer lasting.

### Prophylactic effects of ODN M in the SFFV-mouse model

Next we evaluated the prophylactic effect of ODN M on SFFV replication. For this we established 5 groups of 5 mice each. We pre-incubated SFFV with different ODNs or PBS for 2 hours and then injected the two together intraperitoneally at time 0. Then the mice were treated at 2, 4, and 24 hours post-infection by i.v. route. After 5 days, plasma from the mice was analyzed for viral RNA levels. Mice treated with ODN M (group 3) showed a 5-fold decrease of RNA levels compared to the control mice that were infected and treated with PBS, while mice treated with ODN A showed a 30 % reduction in RNA expression level (Fig. 11 a). A blood smear of the mice at day 64 post-infection was analyzed by Giemsa staining. The blood smear of uninfected and infected ODN M-treated mice showed a normal blood cell population, in contrast to the controls with abnormal blood cell population (data not shown).

We also monitored survival of SFFV-infected mice treated with and without ODN M at times 0 and 1 hour, in this case DBA/2 mice and by i.p. route (Fig. 11b). Untreated mice died from splenomegaly after 20 to 30 days with enlarged spleens of about 2 g. Two out of five ODN M-treated mice survived until more than 60 days. Also these mice died of enlarged spleens reached at the time-point of death. This result indicates that growth of the tumor was delayed and the survival time almost doubled by the initial treatment and reduction of virus infection. We know that disease progression depends on the initial viral titer (data not shown). The spleens of non-infected mice weigh about 0.1 g and are shown in in comparison to the spleen of an infected mouse (data not shown).

In another experimental setting we assessed the effect of an i.v. therapy early after infection. We infected mice with SFFV and treated them 2, 6, and 10 hours later with ODN M, asPPT or PBS. The PCR results obtained from the blood samples show that this treatment prevented the establishment of the SFFV-infection for the whole period of time tested (Fig. 11 c). SFFV-infected mice treated with ODN M or as PPT were healthy, whereas infected and mock-treated mice exhibited an enlargement of the spleens (data not shown).

In order to analyse the efficiency of the ODN A in the vagina, we used a recombinant retrovirus vector (FUGW) and placed the virus with and without ODN A into the vagina. We recover the virus by lavage and tested the RNA copies by real-time PCR. As can be seen (Fig. 12), the virus RNA copies were reduced twofold. Two different virus concentration were used. The effect can be approved due by adaptation to mucosal condition of the vagina.

### DESCRIPTION OF THE DRAWINGS

### Figure 1

(A) The sequences of the extended polypurine tract, PPT, and a site downstream to the PPT of the viral RNA are shown in complex with ODN A and ODN CO accordingly. ODNs consist of an antisense strand and passenger strand linked by four thymidines. Watson-Crick bonds are shown by vertical bars. The sequence of the PPT within the extended PPT is indicated in bold. Antisense oligodeoxynucleotide asEXT targets sites outside of the extended PPT. Relative positioning of ODN A, ODN CO and asEXT on synthetic RNA2 is schematically shown on the lower panel. The box represents the extended PPT and the white stripe symbolises the cleavage site for the RT/RNaseH at the 3' terminus of the PPT within the extended PPT.
(B) The sequences of ODN A and its variants are depicted in hypothetical partially self-complementary hairpin-loop structures. Curved lines symbolize the linker consisting of four phosphorothioated thymidines. ODN Sc has a randomized sequence of both strands and serves as a control for non-specific action of phosphorothioated oligonucleotides. ODN CO targets a region on HIV RNA outside of the PPT. All the other ODNs are designed to target the extended PPT. Abbreviations: Sc - scrambled, B - one nucleotide removed from both 5' and 3' ends of ODN A, T - multiple substitutions in the passenger strand for complete complementarity to the antisense strand, H - triple substitution in the passenger strand for partial complementarity to the antisense strand, NT - triple substitution in the passenger strand in the site complementary to CCCCCC site of the antisense strand, D - single substitution in the passenger strand in the site complementary to AGT site of the antisense strand, CG- single substitution in the passenger strand in the site complementary to the TCT site of the antisense strand. The asEXT was used for primer-extension experiment. Nucleotide changes made compared to ODN A are indicated in bold.

### Figure 2

(A) Cleavage assay. 10 nM of *in vitro* transcribed 5'-labeled RNA was hybridized with 10, 100 and 1000 nM of ODN A and incubated in the presence of RT/RNaseH. The cleavage products were analyzed by denaturing PAGE as described in Materials and methods. (B). Primer extension assay. Synthetic RNA2 was hybridized with ODN A, antisense primer (asEXT) or both primers combined, and the primer extension was performed as described in Materials and Methods. Elongation products were analysed by 8 M urea/10% PAGE. (C) Cleavage assay. RNA/ODN hybrids, obtained using 10 nM of RNA2 and 10 nM of ODN A, asPPT or ODN CO were incubated with RT/RNaseH and cleavage products were analyzed by denaturing PAGE as described in Materials and methods. Cleavage or extension products are presented schematically to the right of each blot. Cleavage sites are indicated by arrows and labelled cleavage products are shown by black lines. Dots on extension products indicate the incorporated labelled dATPs.

### Figure 3

(A) Schematical representation of the sample preparation for real-time PCR
(B) 0.5 µM of ODNs were incubated with intact HIV virions in RPMI cell culture medium for 6 hours and RNA extracted from the treated virions was analyzed by real-time PCR as described in Materials and methods. Each bar represents the mean ±SD of three independent experiments
(C) ODNs do not interfere with RT-PCR reaction. Purified viral RNA was incubated with ODNs in conditions facilitating hybridization. Hybrid complexes were then re-purified and RT-PCR analysis was performed as described in Materials and methods.

### Figure 4

Permeabilized HIV virions were incubated without (A) or with (B) RT/RNaseH (0.05 units/µl), 50 nM ODN A and 150 µM of Illimaquinone, a selective inhibitor of the RNase H activity of HIV RT/RNaseH for 30 min at 37°C. Then viral RNA was purified and real-time PCR analysis was performed. Each bar represents the mean ±SD of three independent experiments.
(C) Intact virions were incubated with 0.5 µM of ODNs for 6 hours in cell culture medium. Total viral RNA was then extracted and analyzed by agarose gel electrophoresis.
(D) Virions were incubated with 0.5 µM of ODNs for 6 hours in cell culture medium. Viral RNA was then extracted, DNase-treated and RT-PCR was performed using primers covering Env-region or PPT-region of HIV RNA. Numbers on the scheme refer to the coordinates of primers on HIV RNA.
(E) Virions containing monodeoxynucleotides were incubated with 0.5 µM of ODNs for 6 hours in cell culture medium to allow primer extension. Viral nucleic acids were then extracted, RNase-treated and PCR was performed using primers specific for viral cDNA and ODN A. 80 bp is the size of the extendes ODN A.

### Figure 5

(A) HIV virions were incubated with 0.5 µM of ODNs for 1, 2 or 4 hours. C81-66/45 cells were infected with pre-incubated virions, at 0.01 MOI. RNA was extracted from infected cells and HIV replication was analysed by real-time PCR, 3 days post-infection (p.i.).
(B) The same experiment as in (A) was performed with ODN T, ODN CO, asPPT and ODN A pre-treated virions.
(C) Comparative efficiency of ODNs in virions. ODNs were incubated with intact HIV virions in cell culture medium for 1, 2 or 4 hours, then the RNA was extracted from the treated virions and analyzed by real-time PCR as described in Materials and methods.
(D) C81-66/45 cells were infected with virions pre-incubated with 0.5 or 5 µM of ODNs for 4h, at an MOI of 1. RNA was extracted from infected cells and HIV replication was analysed by real-time PCR, 3 days post-infection.
(E) C81-66/45 cells were infected with virions pre-incubated with ODN A and ODN Sc for 4h, at an MOI of 0.01. RNA was extracted from infected cells and HIV replication was analysed by real-time PCR, 1 h and 24 h post-infection. (F) The same experiment as in (E) was performed except for using ODN T, ODN B and ODN A, 1 hour post-infection.

### Figure 6

C81-66/45 cells were infected with virions pre-incubated with ODN A, ODN T, ODN CO or as PPT for 4h, at MOI of 0.01. RNA was extracted from infected cells and HIV replication was analysed by real-time PCR, 5 days p.i. (A), and 20 days p.i. (B); by measuring p24 production in the medium of the infected cells (C) or by RT-PCR, 11 days p.i. (D). Each bar represents the mean ±SD of three independent experiments. Syncytia formation is observed in the cells infected with control ODN-treated and not ODN A-treated virions 15 days post-infection (Data not shown).

### Figure 7

a. The SFFV extended PPT sequence with RNase H cleavage site (arrow) is shown. ODNs targeted to the PPT are shown as hairpin-looped structures. Vertical lines indicate base-pairing. The antisense strand (lower strand) is fully complementary to the PPT and linked by four thymidines (curved line) to the passenger strand (upper strand). ODN A targets the PPT of HIV-1 (HIV-1 PPT).
b. Structural organizations of M-MuLV and HIV RT/RNase H are depicted schematically. The 80kDa RT/RNase H of M-MuLV is a monomer while RT/RNase H of HIV-1 is a heterodimer of p66 and p51 and cleaved RNase H.

### Figure 8

a. 5'-labeled synthetic PPT-RNAs of SFFV (left) and HIV-1 (right) were annealed and cleaved with recombinant M-MuLV or HIV-1 RT/RNase H in the absence or presence of ODN M or ODN A. Cleavage products were analyzed by 8M urea-containing PAGE (6 %). Filled arrowheads correspond to the specific RT/RNase H cleavage sites, open arrowheads to additional cleavage sites. RNase T1-digested RNA (RNase T1) served as marker. M indicates the Decade Marker.
b. Titration of recombinant M-MuLV RT/RNase H in the presence of ODN M (50 nM) and 5'-labeled SFFV-PPT-RNA. Increasing amounts of MuLV RT/RNase H (1, 20, and 120 units in 20µl assays) were used (left). 5'-labeled SFFV-PPT-RNA or control RNA without PPT (Con.) were hybridized with the ODNs and treated with and without MuLV RT/RNase H for *in vitro* cleavage as indicated and analyzed by 8M urea-containing PAGE (10%, right).

### Figure 9

a. Cell-free SFFV virions (1x10³ FFU in 20 µl) were permeabilized and incubated with the indicated ODNs (5µM). RNA was extracted three days post-treatment and analyzed by quantitative real-time RT-PCR shown as bars (top) and RT-PCR shown as amplifications products on a 1.5 % agarose gel (bottom).
b. NIH 3T3 cells were infected with SFFV pretreated with various ODNs. After three days the cells and supernatants were harvested and analyzed for SFFV RNA levels by quantitative real time RT-PCR (top) and RT-PCR (bottom) using GAPDH for comparison.
c. NIH-3T3 cells were infected with SFFV for 2 hours and then treated with three decreasing concentrations of ODN M, asPPT and three different PPT-specific siRNAs. RNA was isolated from supernatants three days post-infection and analyzed by real time RT-PCR.

### Figure 10

a. ODN treatment of plasma from SFFV-infected mice *in vivo* and *ex vivo.* Different experimental regimens were utilized for *in vivo* (upper line) and *ex vivo* (lower line) studies. Symbols represent intravenous (i.v.) SFFV infection (↓), mock-infection (⇓), ODN treatment (τ), and bleeding (↓).
   PCR was performed as indicated. 1. Mice infected with 200 µl containing 4x10³ FFU SFFV for 5 days were treated i.v. with ODN M (1mg corresponding to about 20 µM ODN M in the blood of a mouse) or PBS 4 hours post-treatment, plasma (100 µl) was recovered and analyzed by real time RT-PCR for SFFV RNA. 2. Plasma from a 5-day-SFFV-infected mouse was recovered and 100 µl were treated with ODN M (20 µM) for 4 hours at 37 °C *ex vivo* and then analyzed. 3. Plasma from a mock-infected mouse was treated with ODN M (20µM) for 2 hours and then treated with 4 x10³ FFU in 20 µl for 4 hours. 4. Plasma from mock-infected mice was incubated with a combination of SFFV (4x10³ FFU in 20 µl) and ODN M (20 µM) for 4 hours and then analyzed. The RNA levels were determined by real-time RT-PCR in the four experimental scenarios and are shown to the right.
b. Effect of ODN treatment following SFFV-infection *in vivo.* Mice infected with 200 µl containing 4x10³ FFU SFFV for 5 days were treated with ODN M (1mg), asPPT (0.4 mg), ODN A (1mg) or PBS. Real time PCR for SFFV RNA was performed with plasma of the treated mice. 1. Mice were treated with ODNs twice at time 0 and 2h. Viral RNA levels were analyzed at the beginning of the treatment (0h) and 4, 8, and 16 hours post-treatment. 2. Treatment of 5-day-infected mice with ODNs at 0h followed by bleeding at 0, 0.5, 1, 2, and 4 hours. 3. Infected mice were treated twice at two-hour intervals on days 0, 3, and 6 with ODNs. Bleeding was performed before and 4 hours after each double-therapy and SFFV RNA determined. 4. Treatment of 5-day-infected mice with ODNs every 12 hours up to 60 hours. Bleeding was performed prior to treatment.

### Figure 11

a. SFFV virions pre-treated with ODNs for 2 hours were injected i.p. into mice. Subsequent treatments were at 2, 4 and 24h post infection. 5 groups of 5 mice each were used: negative control (1), infection with PBS (2), ODN M (3), or ODN A treatment (4) and mock-infection with ODN M treatment (5). Bleeding was performed five days post-infection for analysis of the viral RNA. The relative RNA levels standardized to group 2 of fully infected mice (bars) as well as RT-PCR products of the individual mice (gel analysis) are shown.
b. Five mice each received 300 µl SFFV (1:100) i.p. supplemented with or without ODN M and 1h later a second dose of ODN M (1 mg each). Survival was monitored until day 68. The spleen weights at death are indicated as numbers on the graph.
c. Mice infected with SFFV (4x10² FFU) i.v. received triple-treatments with ODNs at 2, 6 and 10 hours post-infection. Real-time RT-PCR analysis (graph) and RT-PCR (gel analysis) was performed with RNA from plasma beginning at day 5 and at the days indicated.

### Figure 12

Figure 12 shows the intravaginal treatment of virus with ODN and a subsequent test for inactivation of the virus. The FUGW virus was used, which is a recombinant retrovirus on the basis of HIV but with safety properties. It contains a PPT identical to HIV. The virus was prepared as described (Lois C., Hong E.J., Pease S., Brown E.J. and Baltimore D. (2002). "Germline Transmission and Tissue-Specific Expression of Transgenes Delivered by Lentiviral Vectors", Science 295, 868-872).

To demonstrate the effect of ODN, 20µl containing either 10⁵ Infectious Units, IU, or 10⁴ IU FUGW-virus were used and applied with 25µM ODN for 4 h in the vagina of C57BL/6 mice. The incubation was followed by a lavage of the vagina. Then lavage samples from the vagina were collected for RNA extraction using QIAamp viral RNA kit. RNA were eluted in 30 µl elution buffer. For reverse transcription, 2µl RNA were used in 12.5 µl volume and 3 µl of the cDNA were used for real time PCR. Virus reduction is dependent on the ratio of ODN A to virus titer and the reduction is 2fold for 10⁴ IU.

With respect to mucosal conditions like the secretion in relation to the current hormone status, it is very likely that an optimization of the application form or dose will result in a much higher reduction of virus.

## Claims

1. A pharmaceutical agent comprising an oligodeoxynucleotide (ODN) comprising the sequence of SEQ ID NO: 3 and SEQ ID NO: 4 connected by a linker, which ODN is capable of binding to the extended polypurine tract of HIV-1 for use as a medicament for the protection of a female from HIV-1 infection during sexual intercourse by inactivation of retroviral infectivity outside a cell, wherein the medicament is prepared for application on the vaginal mucosa and inactivation occurs in the vagina.

2. The pharmaceutical agent for use according to at least one of the preceding claims, whereby the ODN comprises SEQ ID NO: 5.

3. The pharmaceutical agent for use according to claim 1, further comprising one or more supplementary agents from the group of antiviral, fungicidal or antibacterial agents, anti-cancer agents and/or immunostimulators or immunomodulators.

4. The pharmaceutical agent for use according to claim 1, wherein the pharmaceutical agent is for the application during sexual intercourse.

## Patentansprüche

1. Ein pharmazeutisches Agens, umfassend ein Oligodeoxynukleotid (ODN), welches die Sequenz von SEQ ID NO: 3 und SEQ ID NO: 4, verbunden durch einen Linker, umfasst, wobei das ODN dazu in der Lage ist, an den erweiterten Polypurin-Trakt von HIV-1 zu binden, zur Verwendung als Medikament für den Schutz einer Frau gegenüber einer HIV-1 Infektion während des Geschlechtsverkehrs durch Inaktivierung der retroviralen Infektivität außerhalb einer Zelle, wobei das Medikament zur Anwendung auf der vaginalen Mukosa zubereitet ist, und wobei die Inaktivierung in der Vagina erfolgt.

2. Das pharmazeutische Agens zur Verwendung nach Anspruch 1, wobei das ODN SEQ ID NO: 5 umfasst.

3. Das pharmazeutische Agens zur Verwendung nach Anspruch 1, ferner umfassend ein oder mehrere zusätzliche Agentien aus der Gruppe von antiviralen, fungiziden oder antibakteriallen Agentien, anti-Tumor-Agentien und / oder Immunostimulatoren oder Immunomodulatoren.

4. Das pharmazeutische Agens zur Verwendung nach Anspruch 1, wobei das pharmazeutische Agens zur Anwendung während des Geschlechtsverkehrs ist.

## Revendications

1. Agent pharmaceutique comprenant un oligodésoxynucléotide (ODN) comprenant les séquences de SEQ ID NO: 3 et de SEQ ID NO: 4 reliées par un lieur, ledit ODN étant capable de se lier à la séquence PPT (Polypurine Tract) étendue du VIH-1 pour son utilisation en tant que médicament pour la protection d'une femme contre une infection par le VIH-1 durant un rapport sexuel par inactivation de l'infectiosité rétrovirale hors d'une cellule, dans lequel le médicament est préparé pour l'application à la muqueuse vaginale et l'inactivation se produit dans le vagin.

2. Agent pharmaceutique pour son utilisation selon au moins l'une des revendications précédentes, dans lequel l'ODN comprend SEQ ID NO : 5.

3. Agent pharmaceutique pour son utilisation selon la revendication 1, comprenant en outre un ou plusieurs agents supplémentaires du groupe des agents antiviraux, fongicides ou antibactériens, des agents anti-cancéreux et/ou des immunostimulateurs ou des immunomodulateurs.

4. Agent pharmaceutique pour son utilisation selon la revendication 1, dans lequel l'agent pharmaceutique est destiné à être appliqué pendant le rapport sexuel.


### SEQUENCE LISTING

<110> Moelling, Karin
<120> Prevention of viral infectivity
<130> XI 1217-06
<150> EP 05090299.8
   <151> 2005-10-27
<150> EP 06090079.2
   <151> 2006-05-12
<150> EP 05090303.8
   <151> 2005-11-03
<160> 43
<170> PatentIn version 3.3
<210> 1
   <211> 25
   <212> DNA
   <213> HIV-1 PPT DNA
<900> 1
   aaaagaaaag gggggactgg aaggg 25
<210> 2
   <211> 25
   <212> DNA
   <213> Artificial
<220>
   <223> synthetic oligodeoxynucleotide
<400> 2
   ttttgttttg gggggtgtgg ttggg 25
<210> 3
   <211> 25
   <212> DNA
   <213> ODN A 3' part, PPT (asPPT)
<400> 3
   cccttccagt cccccctttt ctttt 25
<210> 4
   <211> 25
   <212> DNA
   <213> ODN A 5' part, PPT
<400> 4
   ttttcttttg gggggtttgg ttggg 25
<210> 5
   <211> 54
   <212> DNA
   <213> ODN A
<400> 5
   ttttcttttg gggggtttgg ttgggttttc ccttccagtc cccccttttc tttt 54
<210> 6
   <211> 54
   <212> DNA
   <213> ODN D
<400> 6
   ttttcttttg gggggtctgg ttgggttttc ccttccagtc cccccttttc tttt 54
<210> 7
   <211> 54
   <212> DNA
   <213> ODN A(C-T)
<400> 7
   tttttttttg gggggtttgg ttgggttttc ccttccagtc cccccttttc tttt 54
<210> 8
   <211> 54
   <212> DNA
   <213> ODN A(C-G)
<400> 8
   ttttgttttg gggggtttgg ttgggttttc ccttccagtc cccccttttc tttt 54
<210> 9
   <211> 24
   <212> DNA
   <213> ODN B 3' part
<400> 9
   cccttccagt cccccctttt cttt 24
<210> 10
   <211> 24
   <212> DNA
   <213> ODN B 5' part
<400> 10
   tttcttttgg ggggtttggt tggg 24
<210> 11
   <211> 52
   <212> DNA
   <213> ODN B
<400> 11
   tttcttttgg ggggtttggt tgggttttcc cttccagtcc ccccttttct tt 52
<210> 12
   <211> 25
   <212> DNA
   <213> asPPT
<400> 12
   cccttccagt cccccctttt ctttt 25
<210> 13
   <211> 54
   <212> DNA
   <213> ODN SC
<400> 13
   tttggggggt tcttcctcct ttcctttttc gcccgtccgt tgcgttgatt tttt 54
<210> 14
   <211> 54
   <212> DNA
   <213> ODN T
<400> 14
   aaaagaaaag gggggactgg ttgggttttc ccttccagtc cccccttttc tttt 54
<210> 15
   <211> 54
   <212> DNA
   <213> ODN H
<400> 15
   ttttcttttg gggggactgg ttgggttttc ccttccagtc cccccttttc tttt 54
<210> 16
   <211> 54
   <212> DNA
   <213> ODN NT
<400> 16
   ttttcttttg cgcgctttgg ttgggttttc ccttccagtc cccccttttc tttt 54
<210> 17
   <211> 54
   <212> DNA
   <213> ODN D
<400> 17
   ttttcttttg gggggtctgg ttgggttttc ccttccagtc cccccttttc tttt 54
<210> 18
   <211> 48
   <212> DNA
   <213> ODN CG
<400> 18
   tctttttgcg cgctttgttt gttttttctt tcattccccc ctttttct 48
<210> 19
   <211> 17
   <212> DNA
   <213> asEXT
<400> 19
   catgcacgtg tgacgtt 17
<210> 20
   <211> 48
   <212> DNA
   <213> ODN M
<400> 20
   tctttttggg gggtttgttt gttttttctt tcattccccc ctttttct 48
<210> 21
   <211> 48
   <212> DNA
   <213> ODN F
<400> 21
   agaaaaaggg gggaatgaaa gatttttctt tcattccccc ctttttct 48
<210> 22
   <211> 48
   <212> DNA
   <213> ODN S
<400> 22
   tctttttggg gggaatgttt gttttttctt tcattccccc ctttttct 48
<210> 23
   <211> 22
   <212> DNA
   <213> ODN M(AS)
<400> 23
   tctttcattc cccccttttt ct 22
<210> 24
   <211> 25
   <212> DNA
   <213> synthetic
<220>
   <221> DNA
   <222> (6)..(11)
<400> 24
   cccuuccagt ccccccuuuu cuuuu 25
<210> 25
   <211> 25
   <212> DNA
   <213> synthetic
<220>
   <221> DNA
   <222> (8)..(12)
<400> 25
   cccuuccagt ccccccuuuu cuuuu 25
<210> 26
   <211> 25
   <212> DNA
   <213> synthetic
<220>
   <221> DNA
   <222> (1) .. (7)
<220>
   <221> DNA
   <222> (13)..(25)
<400> 26
   cccttccagu cccccctttt ctttt 25
<210> 27
   <211> 21
   <212> DNA
   <213> siRNA 5'part
<220>
   <221> DNA
   <222> (20)..(21)
   <223> part of the T4 linker
<400> 27
   agaaaagggg ggacuggaat t 21
<210> 28
   <211> 21
   <212> DNA
   <213> siRNA 3'part
<220>
   <221> DNA
   <222> (20)..(21)
   <223> part of the T4 linker
<400> 28
   uuccaguccc cccuuuucut t 21
<210> 29
   <211> 25
   <212> RNA
   <213> 3-PPT of HIV-IIIB RNA
<400> 29
   aaaagaaaag gggggacugg aaggg 25
<210> 30
   <211> 24
   <212> RNA
   <213> 5-PPT of HIV-IIIB RNA (internal PPT)
<400> 30
   aaaagaaaag gggggauugg gggg 24
<210> 31
   <211> 37
   <212> RNA
   <213> Primer binding site (PBS) of HIV
<400> 31
   aguggcgccc gaacagggac ugaaagcgaa agggaaa 37
<210> 32
   <211> 32
   <212> RNA
   <213> poly (A)-tail
<400> 32
   aaaaaaaaaa aaaaaaaaaa aaaaaaaaaa aa 32
<210> 33
   <211> 32
   <212> RNA
   <213> PPT of HIV Bal
<400> 33
   uuuaagaaaa aggggggacu ggaagggcuu aa 32
<210> 34
   <211> 22
   <212> RNA
   <213> PPT of SFFV
<400> 34
   agaaaaaggg gggaaugaaa ga 22
<210> 35
   <211> 25
   <212> RNA
   <213> 3-PPT of HIV variants
<400> 35
   aaaagaaaag gggggacugg aaggg 25
<210> 36
   <211> 25
   <212> RNA
   <213> 3-PPT of HIV variants
<400> 36
   aagagaaaag gggggacugg aaggg 25
<210> 37
   <211> 25
   <212> RNA
   <213> 3-PPT of HIV variants
<400> 37
   aaaagaaaag gggggacugg auggg 25
<210> 38
   <211> 25
   <212> RNA
   <213> 3-PPT of HIV variants
<400> 38
   aaaagaagag gggggacugg aaggg 25
<210> 39
   <211> 25
   <212> RNA
   <213> 3-PPT of HIV variants
<400> 39
   aaaagaacag gggggacugg aaggg 25
<210> 40
   <211> 25
   <212> RNA
   <213> 3-PPT of HIV variants
<400> 40
   uaagaaaaag gggggacugg aaggg 25
<210> 41
   <211> 28
   <212> RNA
   <213> Hepatitis B Virus RNA
<400> 41
   aaagacuggg aggaguuggg ggaggaga 28
<210> 42
   <211> 34
   <212> RNA
   <213> PSI HIV RNA
<400> 42
   gaggcgaggg gcggcgacug gugagacgcc aaaa 34
<210> 43
   <211> 28
   <212> RNA
   <213> Epsilon HBV RNA
<400> 43
   ccguguuggg ggcuuugggg cauggaca 28